# EUROPEAN PATENT SPECIFICATION

(11) **EP 1 082 970 B1**
(45) Date of publication and mention of the grant of the patent: **16.08.2006**
(21) Application number: 99830557.7
(22) Date of filing: 07.09.1999
(51) Int. Cl.: A61L 9/12

(54) **Container for volatile essences**
Behälter zur Abgabe flüchtiger Duftstoffe
Réservoir permettant la libération des essences volatiles

(43) Date of publication of application: 14.03.2001
(73) Proprietor: General FIX S.R.L., 20090 Opera (Milano) (IT)
(72) Inventor: Azzolin, Itala, 20085 Locate Triulzi (Milano) (IT)
(74) Representative: Petruzziello, Aldo

(56) References cited:
- EP-A- 0 302 471
- WO-A-97/22370
- WO-A-98/00179
- GB-A- 2 158 356
- US-A- 4 254 910
- US-A- 5 788 061

## Description

The present invention relates to a container for substances that volatilize, in liquid, solid or gel form, such as fragrances, deodorants, moth-repellents and the like. Said container is particularly suitable to be used in wardrobes, drawers, shoe cupboards and small or large rooms, according to the size of the relative container.

Volatile substances, such as perfumed essences, are generally packaged in bottles, plastic containers or impregnated cards.

For reasons of economy of manufacture, size and practicality of use by the consumer, small containers with membranes that are permeable or semi-permeable to vapour, capable of releasing the essence slowly into the surrounding environment have come into wide use. In this type of container the permeable membrane is normally coated with a peelable protection that prevents escape of the volatile essence before use.

This type of container according to the prior art has drawbacks related above all to practical difficulties in removing said peelable protection and to the fact that during use, for example in a wardrobe or in a drawer, the permeable membrane can stain the fabric of clothes with which it comes into contact.

US patent 4.254.910 describes a package for controlled release of volatile substances, according to the preamble of claim 1. Said package comprises a pair of half-shells, made of impermeable plastic, suitable for containing volatile substances. Said half-shells are symmetrically arranged one against the other, separated by an intermediate layer of vapour-permeable material. For use of the container the two half-shells are separated by pulling apart the intermediate layer, which separates into two vapour-permeable sheets, one of which remains attached to each half-shell. In this manner two individual containers are obtained that can be used separately, for each of which the above mentioned problems due to the possibility of soiling articles of clothing with which the permeable membrane comes into contact remain.

The object of the invention is to eliminate said drawbacks by providing a container for volatile essences that is practical, versatile, simple to make and prevents direct contact of its permeable membrane with items such as articles of clothing.

Another object of the invention is to provide such a container that is able to stand alone in an upright position on a surface.

Another object of the invention is to provide a container that is able to contain two different types of volatile substances.

These objects are achieved according to the invention with the container according to appended independent claim 1 and with the manufacturing method for the container according to appended independent claim 8.

Preferred embodiments of the invention are apparent from the dependent claims.

The container for volatile essences according to the invention is obtained from a single outer layer of rigid plastic material, impermeable to vapour, from which a pair of half-shells, comprising respective cups destined to contain volatile substances and surrounded by respective external frames, is obtained by heat moulding.

The outer layer is folded along a median line so that the two half-shells can be disposed symmetrically one against the other. An intermediate layer formed by a vapour-permeable membrane is inserted between the two half-shells. The intermediate layer is then fixed to the frames of the two half-shells by heat moulding, maintaining the container in a closed position.

Two opposite terminal flaps of the container are not heat sealed and remain spaced apart from each other to allow gripping by the user.

In order to open the container the user grips the two free terminal flaps and pulls, causing tearing of the intermediate permeable layer that divides into two permeable sheets, each fixed to the respective half-shell.

In this manner the container opens like a book, forming an upturned V-shaped structure that stands alone on the end flaps so as to be able to be positioned upright inside drawers or on shelves and the vapour of the volatile essence is thus free to diffuse into the outer environment through the permeable membranes that cover the respective half-shells.

The container according to the invention, in its open configuration, has the impermeable layer facing outward, whereas the permeable membranes face inward; thus even if the outer part of the container should come into contact with articles of clothing there is no danger of staining them through escape of the essence contained in the container.

The container according to the invention further provides the possibility of filling the cups of the half-shells that compose it with two different essences that give off two different fragrances and perform two different functions, for example, deodorant and moth-repellent, or said cups can be filled with essences of different colours to obtain a particular decorative effect.

Further characteristics of the invention will by made clearer by the detailed description that follows, referring to a purely exemplary and therefore non limiting embodiment thereof, illustrated in the appended drawings, in which:
Figure 1 is a plan view of the container for volatile essences according to the invention in its closed configuration;
Figure 2 is a view from the right or left side of the container in Figure 1;
Figure 3 is a cross section of the closed container, along the plane III-III in Figure 1;
Figure 4 is a perspective view of the container according to the invention, open and in the upright position;
Figure 5 is a cross section, like Figure 3, of the container in the open configuration.

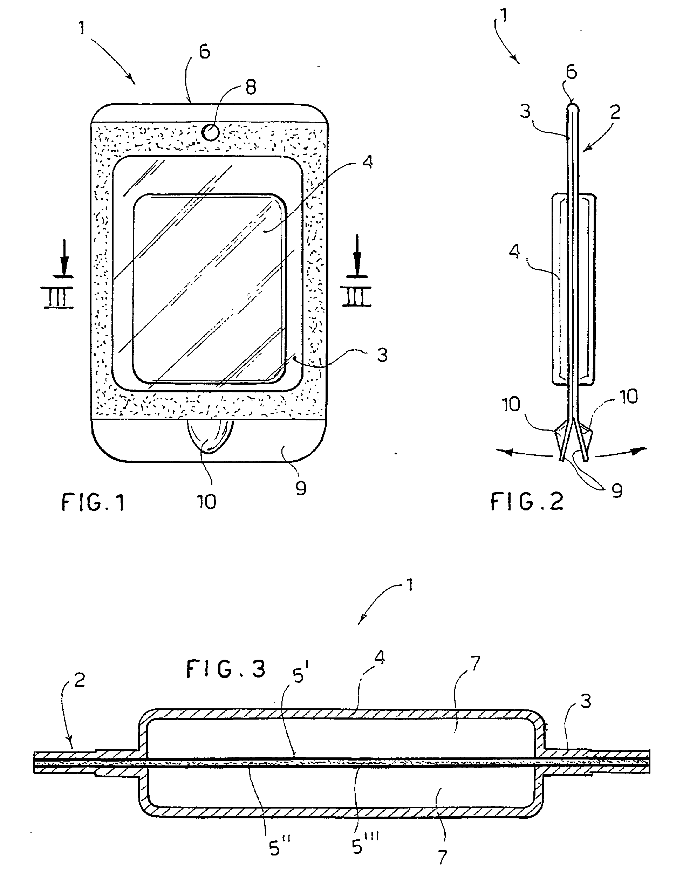

Figures 1-3 show a container for volatile essences according to the invention, in closed configuration, denoted as a whole with reference numeral 1.

The container 1 comprises an outer layer 2 in gas-impermeable material, such as polyester for example, in particular PET (polyethylene terephthalate), which forms a barrier to release of volatile essences.

The outer layer 2 is heat moulded so as to obtain a pair of half-shells. Each half-shell comprises a frame, rectangular in the example shown, surrounding a flattened cup 4.

The outer layer 2 is folded along a median edge line 6 which symmetrically divides the two half-shells. In this manner the two half-shells are disposed one against the other with the bottom of each cup 4 facing outward.

Sandwiched between the two half-shells is an intermediate layer 5, which can be made by means of single-layer or multilayer material having a certain gas-permeability, so as to allow slow release of the essences contained in each half-shell. In this manner a chamber 7 destined to contain a volatile essence is formed between the intermediate layer 5 and each cup 4 of each half-shell.

The intermediate layer of material 5 comprises two outer layers 5', 5", which can be heat sealed to the half-shells, joined by a separable central layer 5"', consisting of paper or permeable sheets that can be peeled away from each other. The outer layers 5', 5" are obviously permeable and have at least the surface that comes into contact with the half-shells of polyethylene, for example, so that it can be heat sealed to the half-shells.

Once the intermediate layer 5 has been disposed between the two half-shells, a heat seal is made along the frame 3 of the outer layer 2, with the exception of a small central portion of the side of the frame opposite the fold line 6. Heat sealing allows the intermediate layer 5 to be fixed to the frame 4 and the container 1 is thus maintained in a closed configuration.

As shown in Figure 2, the part of frame 3 opposite the fold line 6 ends in two flaps 9 spaced slightly apart from each other and opening in a dovetail for easier gripping by the user.

For filling of the container 1, seats 10 able to receive respective nozzles for injection of the volatile material into the chambers 7 are provided in the terminal flaps 9.

After filling of the chambers 7 of the pair of half-shells a heat seal is made along the remaining strip of frame 3 comprised between the terminal edges 9 and the cups 4. In this manner the container 1 is perfectly closed and sealed, excluding any possibility of escape of the vapours given off by the volatile substances contained in the chambers 7.

In the part of frame 3 beneath the fold line 6, a through hole 8 is provided such as to accommodate a thong or a hook to allow the container 1 to be hung up.

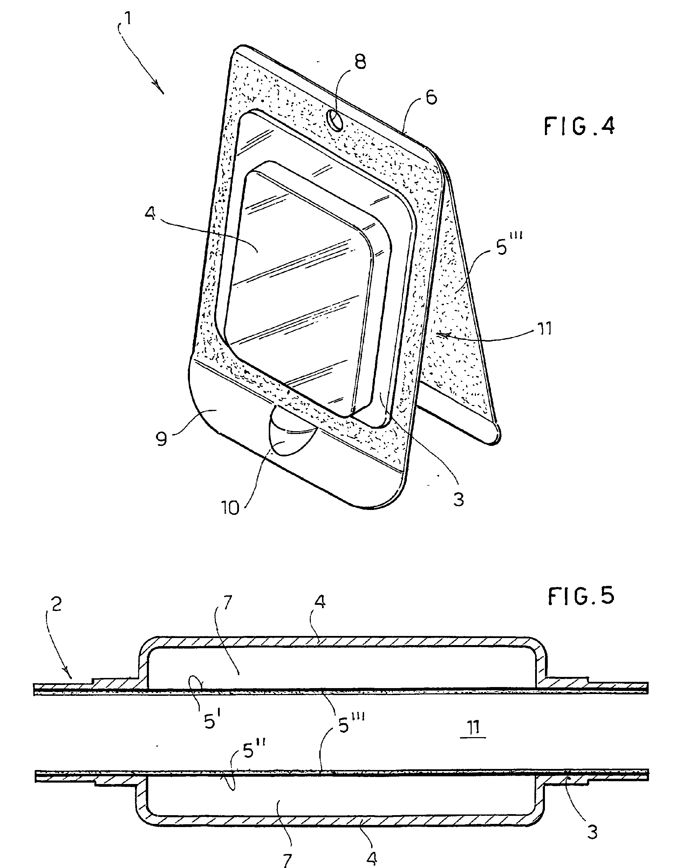

In order to open the container 1, the user must simply grip the end flaps 9 and exert a certain pull. This parts the central layer 5"' of the intermediate layer 5 which, as shown in Figure 5, is divided into two thinner layers, which remain attached, together with their respective permeable membranes 5' 5", to the respective frames 3 of the two half-shells of the container 1.

The container 1 therefore opens like a book, taking on an upturned V shape, and the two half-shells remain joined only along the fold line 6. The permeable layers 5', 5" are spaced apart from each other so that an air space 11 is formed therebetween.

On resting the end flaps 9 on a surface, the container 1 is able to stand alone, in an upright position, in an upturned V shape configuration. In this open configuration the essence contained in the chamber 7 passes through the permeable layers 5', 5" and the portion of central layer 5"', also permeable, and volatilises in the air space 11, diffusing into the surrounding environment.

It is apparent that when the container 1 is in the open configuration, both in the case of it being hung by a hook or a thong engaged in the hole 8, and the case of it being rested on a surface, the permeable membranes 5', 5" cannot come into contact with articles of clothing or other objects and soil because of escape of the essence. In fact, when the container according to the invention is in said open configuration the only part that can come into contact with articles of clothing or objects is the outer layer 2 which is impermeable and does not allow the essence to escape.

The container 1 according to the invention, further providing a pair of chambers 7 to contain the volatile essences, proves particularly suitable should it be wished to fill the two chambers 7 with two essences of different types. The chambers 7 can be filled with two different fragrances, for example, or with a fragrance and a moth repellent, or whatever else.

The outer layer 2 that forms the pair of cups 4 can advantageously be made of transparent material, thus in this case the two chambers 7 can be filled with essences of different colours for decorative reasons.

Modifications within the reach of a skilled in the art can be made to the above described embodiment of the invention. For example, instead of using heat sealing to fix the intermediate layer 5 to the frame 3, adhesive means or other known methods can be used.

Otherwise the container according to the invention, instead of with a single outer layer 2, can be made with two separate half-shells that are held together along the edge line 6 by known methods or fixing means.

## Claims

1. A container for volatile essences comprising a vapour-impermeable outer layer (2) forming two opposite half-shells, each comprising a respective cup (4) to contain said volatile essence, and an outer frame (3), a separable intermediate layer (5), permeable to vapours, being disposed between the two half-shells and joined to the half-shells along said outer frames (3), **characterized in that** said vapour-impermeable half-shells are made from a single outer layer (2) folded along an edge line (6), situated in a symmetrical position between the two half-shells so that said container (1) can be opened like a book by parting of said intermediate permeable layer (5) and can stand alone in an upright position.

2. A container according to claim 1, **characterized in that** said intermediate permeable layer (5) comprises two outer layers (5', 5") heat sealed to the respective half-shells of the container and a separable central layer (5"'):

3. A container according to claim 2, **characterized in that** said central layer (5"') is paper.

4. A container according to claim 2, **characterized in that** said central layer (5 "') comprises peelable sheets of permeable material.

5. A container according to any one of the preceding claims, **characterized in that** two end flaps (9) are provided in said outer layer (2), said flaps being spaced apart to allow gripping by the user for opening of the container (1) and able to support the open container (1) in an upright position.

6. A container according to claim 5, **characterized in that** seats (10) are made in said end flaps such as to accommodate nozzles for injection of volatile essences during the manufacturing process of the container (1).

7. A container according to any one of the preceding claims, **characterized in that** a hole (8) is made in said outer layer (2) such as to accommodate means for hanging said container (1).

8. A manufacturing method for a container for volatile essences comprising the following steps:
heat moulding of an outer layer (2) of rigid plastic material for the formation of a pair of half-shells;
folding of said outer layer (2) along an edge line (6) situated in a symmetrical position between the two half-shells;
insertion of an intermediate layer (5) of vapour-permeable material between said half-shells so as to form a pair of chambers (7) to contain the volatile material;
heat sealing of said intermediate layer (5) along an edge region of said half-shells of the outer layer (2);
injection filling of said pair of chambers (7);
heat sealing of the part of the container (2) left open for insertion of the injection nozzles.

## Patentansprüche

1. Ein Behälter für flüchtige Essenzen, der eine dampfundurchlässige äußere Schicht (2) umfasst, die zwei gegenüberliegende Halbschalen bildet, wobei jede jeweils ein Gefäß (4) umfassen, um die genannte flüchtige Essenz zu enthalten, und ein äußeres Gestell (3), eine trennbare Zwischenschicht (5), die dampfdurchlässig ist, zwischen den beiden Halbgehäusen angeordnet und mit den Halbgehäusen entlang den genannten äußeren Gestellen (3) verbunden ist, **dadurch gekennzeichnet, dass** die genannten dampfundurchlässigen Halbschalen aus einer einzigen äußeren Schicht (2) bestehen, die entlang einer Kantenlinie (6) gefalzt ist, welche sich in symmetrischer Lage zwischen den beiden Halbschalen befindet, damit der genannte Behälter (1) wie ein Buch durch Auseinandergehen der genannten durchlässigen Zwischenschicht (5) getrennt werden und allein in aufrechter Stellung stehen kann.

2. Ein Behälter gemäß Anspruch 1, **dadurch gekennzeichnet, dass** die genannte durchlässigen Zwischenschicht (5) zwei äußere Schichten (5', 5") umfasst, die mit den entsprechenden Halbschalen des Behälters und einer trennbaren mittleren Schicht (5"') verschweißt sind.

3. Ein Behälter gemäß Anspruch 2, **dadurch gekennzeichnet, dass** die genannte mittlere Schicht (5"') aus Papier besteht.

4. Ein Behälter gemäß Anspruch 2, **dadurch gekennzeichnet, dass** die genannte mittlere Schicht (5'") jeweils abziehbare Folien aus durchlässigem Material umfasst.

5. Ein Behälter gemäß einem beliebigen der vorausgegangenen Ansprüche, **dadurch gekennzeichnet, dass** zwei Endlaschen (9) in genannten äußeren Schicht (2) vorgesehen sind, wobei die genannten Laschen so auseinander liegen, dass sie vom Benutzer jeweils zur Öffnung des Behälters (1) erfasst werden können, und in der Lage sind, den offenen Behälter (1) in aufrechter Stellung zu halten.

6. Ein Behälter gemäß Anspruch 5, **dadurch gekennzeichnet, dass** sich in den genannten Endlaschen Sitze (10) befinden, so dass während des Fertigungsverfahrens des Behälters (1) jeweils Einspritzdüsen für die flüchtigen Essenzen untergebracht werden können.

7. Ein Behälter gemäß einem beliebigen der vorausgegangenen Ansprüche, **dadurch gekennzeichnet, dass** eine Öffnung (8) sich in der der genannten äußeren Schicht (2) befindet, so dass Elemente zum Aufhängen des genannten Behälters (1) angebracht werden können.

8. Eine Herstellungsmethode für einen Behälter für flüchtige Essenzen, die folgende Schritte umfasst:
- Formen einer äußeren Schicht (2) von starrem Kunststoffmaterial für die Bildung eines Paars von Halbschalen;
- Falzen der genannten äußeren Schicht (2) entlang einer Kantenlinie (6), die sich in symmetrischer Lage zwischen den beiden Halbschalen befindet;
- Einsatz einer Zwischenschicht (5) aus dampfdurchlässigem Material zwischen den genannten Halbschalen, um ein Paar Kammern (7) zu bilden, die das flüchtige Material enthalten;
- Verschweißen der genannten Zwischenschicht (5) entlang des Kantenbereichs der genannten Halbschalen der äußeren Schicht (2);
- Füllung des genannten Kammerpaars (7) per Einspritzung;
- Verschweißen des Teil des Behälters (2), der offen gelassen wurde für den Einsatz der Einspritzdüsen.

## Revendications

1. Récipient pour essences volatiles comprenant une couche extérieure imperméable à la vapeur (2) formant deux demi-coquilles opposées, chacune comprenant une tasse respective (4) pour contenir ladite essence volatile, et une structure extérieure (3), une couche intermédiaire séparable (5), perméable aux vapeurs, étant placée entre les deux demi-coquilles et reliée aux demi-coquilles le long desdites structures extérieures (3), **caractérisé en ce que** lesdites demi-coquilles imperméables à la vapeur sont réalisées dans une seule couche extérieure (2) pliée le long d'un ligne de bordure (6), placée dans une position symétrique entre les deux demi-coquilles de sorte que ledit récipient (1) peut être ouvert comme un livre en séparant en deux ladite couche intermédiaire perméable (5) et qu'il peut se soutenir seul dans une position droite.

2. Récipient selon la revendication 1, **caractérisé en ce que** ladite couche perméable intermédiaire (5) comprend deux couches extérieures (5', 5") thermosoudées sur les demi-coquilles respectives du récipient et une couche centrale séparable (5"').

3. Récipient selon la revendication 2, **caractérisé en ce que** ladite couche centrale (5"') est du papier.

4. Récipient selon la revendication 2, **caractérisé en ce que** ladite couche centrale (5"') comprend des feuilles détachables de matériau perméable.

5. Récipient selon une revendication quelconque parmi les revendications susmentionnées, **caractérisé en ce que** deux volets d'extrémité (9) sont prévus dans ladite couche extérieure (2), lesdits volets étant espacés pour permettre à l'utilisateur de les saisir pour ouvrir le récipient (1) et en mesure de soutenir le récipient en ouverture (1) dans une position droite.

6. Récipient selon la revendication 5, **caractérisé en ce que** des logements (10) sont réalisés dans lesdits volets d'extrémité de façon à accueillir des buses d'injection d'essences volatiles pendant le processus de fabrication du récipient (1).

7. Récipient selon une revendication quelconque parmi les revendications susmentionnées, **caractérisé en ce qu'**un orifice (8) est réalisé dans ladite couche extérieure (2) de façon à accueillir des dispositifs d'accrochage dudit récipient (1).

8. Méthode de fabrication d'un récipient pour essences volatiles comprenant les phases suivantes :
thermo-moulage d'une couche extérieure (2) en matière plastique rigide pour la formation d'une paire de demi-coquilles ;
pliage de ladite couche extérieure (2) le long d'une ligne de bordure (6) placée dans une position symétrique entre les deux demi-coquilles ;
introduction d'une couche intermédiaire (5) de matière perméable à la vapeur entre lesdites demi-coquilles de façon à former une paire de chambres (7) pour contenir la matière volatile ;
thermo-soudure de ladite couche intermédiaire (5) le long d'une région de bordure desdites demi-coquilles de la couche extérieure (2) ;
remplissage par injection de ladite paire de chambres (7) ;
thermo-soudure de la partie du récipient (2) restée ouverte pour l'introduction de buses à injection.
